# FASCICULE DE BREVET EUROPEEN

(11) **EP 2 252 709 B1**
(45) Date de publication et mention de la délivrance du brevet: **12.12.2012**
(21) Numéro de dépôt: 09726540.9
(22) Date de dépôt: 09.03.2009
(51) Int. Cl.: C12Q 1/68, C07H 21/00

(54) **METHODE, PROCEDE, ET UTILISATION DE DIAGNOSTIC, PRONOSTIC, SUIVI D'UN CANCER BASES SUR L'ARN EXTRACELLULAIRE DE CA9**
VERFAHREN, PROZESS UND KIT ZUR KREBSDIAGNOSE ODER -PROGNOSE UND -NACHBEHANDLUNG AUF BASIS VON EXTRAZELLULAREN CA9-RNA
METHOD, PROCESS, AND USES FOR CANCER DIAGNOSIS, PROGNOSIS, AND AFTERCARE BASED ON EXTRACELLULAR RNA OF CA9

(30) Priorité: 14.03.2008 FR 0801401
(43) Date de publication de la demande: 24.11.2010
(73) Titulaire: Chu Saint Etienne Chu - Hopital Nord, 42270 Saint Priest En Jarez (FR); Université Jean-Monnet, 42023 Saint-Etienne Cedex 2 (FR)
(72) Inventeur: LI, Guorong, 42100 Saint Etienne (FR); TOSTAIN, Jacques, 42580 L' Etrat (FR); GENIN, Christian, 42000 Saint Etienne (FR)
(74) Mandataire: Aupetit, Muriel J. C.
(86) Numéro de dépôt international: PCT/FR2009/050381
(87) Numéro de publication internationale: WO 2009/122051

(56) Documents cités:
- EP-A- 1 595 947
- EP-A- 1 712 639
- WO-A-03/089659
- WO-A-2006/061216
- WO-A-2007/098444
- FENG G ET AL: "CIRCULATING CA9 AND S100A1 DNA IN SERUM FOR MOLECULAR DIFFERENTIAL DIAGNOSIS OF RENAL TUMOURS" EUROPEAN UROLOGY SUPPLEMENTS, vol. 7, no. 3, 12 mars 2008 (2008-03-12), page 97, XP022610107 ISSN: 1569-9056
- MCKIERNAN JAMES M ET AL: "The detection of renal carcinoma cells in the peripheral blood with an enhanced reverse transcriptase-polymerase chain reaction assay for MN/CA9" CANCER, vol. 86, no. 3, 1 août 1999 (1999-08-01), pages 492-497, XP002497577 ISSN: 0008-543X
- GUORONG LI ET AL: "CA9 gene expression in conventional renal cell carcinoma: a potential marker for prediction of early metastasis after nephrectomy" CLINICAL & EXPERIMENTAL METASTASIS, vol. 24, no. 3, 28 mars 2007 (2007-03-28), pages 149-155, XP019499423 ISSN: 1573-7276
- LI ET AL: "The Use of MN/CA9 Gene Expression in Identifying Malignant Solid Renal Tumors" EUROPEAN UROLOGY, ELSEVIER BV, NL, vol. 49, no. 2, 1 février 2006 (2006-02-01), pages 401-405, XP005426449 ISSN: 0302-2838
- FENG GANG ET AL: "Elevated serum-circulating RNA in patients with conventional renal cell cancer." ANTICANCER RESEARCH 2008 JAN-FEB, vol. 28, no. 1A, janvier 2008 (2008-01), pages 321-326, XP008096889 ISSN: 0250-7005
- FLEISCHHACKER M ET AL: "Circulating nucleic acids (CNAs) and cancer--a survey." BIOCHIMICA ET BIOPHYSICA ACTA JAN 2007, vol. 1775, no. 1, janvier 2007 (2007-01), pages 181-232, XP002497578 ISSN: 0006-3002
- LI ET AL: "MN/CA9 as a novel molecular marker for the detection of cancer" EXPERT OPINION ON MEDICAL DIAGNOSTICS, vol. 1, no. 1, septembre 2008 (2008-09), pages 91-97, XP008096872
- HULICK P. ET AL: "Blood Levels of Carbonic Anhydrase 9 Correlate with Clear Cell Renal Cell Carcinoma Activity" CLINICAL PROTEOMICS, 12 juillet 2008 (2008-07-12), pages 1-9, XP002497579 ISSN: 1559-0275

### SEQUENCE LISTING

<110> CHU Saint Etienne and Université Jean Monnet
<120> Method and kit for diagnosis/prognosis of cancer
<160> 6
<210> 1
   <211> 20
   <212> RNA
   <213> vertebrate
<400> 1
   GGA CAA AGA AGG GGA TGA CC
   20
<210> 2
   <211> 19
   <212> RNA
   <213> vertebrate
<400> 2
   AGT TCT GGG AGC GGC GGG A
   19
<210> 3
   <211> 21
   <212> RNA
   <213> vertebrate
<400> 3
   CAG AGT CAT TGG CGC TAT GGA
   21
<210> 4
   <211> 17
   <212> RNA
   <213> vertebrate
<400> 4
   CGA GCT GGG GGC GGA TA
   17
<210> 5
   <211> 186
   <212> RNA
   <213> vertebrate
<400> 5
<210> 6
   <211> 100
   <212> RNA
   <213> vertebrate
<400> 6

## Description

La présente invention concerne une méthode ou procédé et des utilisations permettant la mise en évidence précoce d'un cancer, en vue de son diagnostic, son pronostic ou lors de son suivi thérapeutique.

Plus particulièrement, la présente invention se rapporte à une nouvelle méthode, aux amorces d'amplification et aux sondes d'hybridation qui peuvent être mises en oeuvre dans ce procédé, ainsi qu'à des utilisations aux fins de diagnostic/pronostic/suivi d'un cancer chez les vertébrés.

Un «diagnostic» se définit comme la détermination d'une affection d'une personne atteinte d'une pathologie donnée ; un «pronostic» comme le degré de gravité permettant de prévoir l'évolution ultérieure d'une pathologie, et «thérapeutique» signifie un traitement à visée curative ou palliative proposé à un individu.

Le plus souvent un cancer rénal, par exemple, est asymptomatique et le diagnostic se réalise via des examens radiologiques tels une échographie, tomodensimétrie (scanner), urographie, ou imagerie par résonance magnétique (IRM) etc.

Cependant aux stades très précoces, il est difficile de détecter les petites tumeurs et d'affirmer leur nature cancéreuse par l'imagerie. La répétition des examens d'imagerie et/ou un examen histologique ou cytologique après biopsie ou ponction seront nécessaires.

Ces examens sont coûteux, manquent de sensibilité et certains prélèvements biologiques sont très invasifs et/ou douloureux.

II existe des tests de biologie moléculaire axés sur la recherche de l'ARN messager (ARNm) ou le dosage de la protéine d'un gène MN/CA9 présent dans les cellules malignes d'un individu atteint d'un cancer.

Le MN/CA9 est un marqueur moléculaire efficace pour la détection des cellules cancéreuses. Il est également appelé anhydrase carbonique 9 ou CA9. Ce gène encode une oncoprotéine antigène de membrane MN, l'isoenzyme MN/CA9 (genebank accession number : NM 000009). Le gène CA9 a pour fonction principale de réguler le pH et il est exprimé dans de nombreux cancers, et plus particulièrement dans le cancer du rein, notamment dans le type de tumeur le plus fréquent, le Carcinome Rénal à Cellules Conventionnelles (CRCC).

Les recherches actuelles tentent de mettre en évidence et de quantifier les ARNm des gènes impliqués dans un cancer dans les cellules tumorales circulantes, à partir d'extraits de cellules, ou la présence de la protéine CA9 dans un échantillon biologique.

Cependant ces études sont limitées soit aux échantillons prélevés dans une tumeur soit à une sensibilité très faible dans le sang des individus cancéreux. Ces tests ne sont donc pas toujours pertinents en tant que test de diagnostic.

De plus, ces tests ne permettent pas de dépister des stades précoces lors du diagnostic initial ou lors d'une récidive.

La demande de brevet américain n°US2007224606 se ra pporte à une méthode de pronostic comprenant la détection de la présence des produits d'expression du gène CA9. Il y est divulgué une corrélation de l'expression du gène CA9 avec le pronostic du cancer du rein. Cependant, la valeur seuil utilisée correspond à 50% de cellules positives dans un échantillon de tumeur.

La demande de brevet internationale n° W00110910, montre une méthode permettant d'utiliser la protéine d'expression du gène CA9 comme marqueur en vue d'améliorer le diagnostic cytologique.

Feng Gang et al (Elevated serum-circulating RNA in patients with conventional renal cell cancer, ANTICANCER RESEARCH vol. 28, no. 1 A, janvier 2008, pages 321-326) décrit une méthode pour le diagnostic du carcinome rénal à cellules conventionnelles (CRCC, ou RCC) chez les vertébrés, comprenant l'extraction d'ADN extracellulaire à partir de sérum et l'amplification par PCR quantitative de l'ADN du gène CA9.

Le procédé selon la présente invention permet notamment de déterminer la présence ou non d'un cancer, d'évaluer sa gravité, de déterminer le traitement le plus adapté ainsi que d'assurer le suivi des individus traités.

La présente invention pallie les inconvénients de l'art antérieur en proposant une nouvelle méthode, un procédé et des utilisations permettant la mise en évidence d'un cancer, en vue du diagnostic, du pronostic ou de son suivi thérapeutique.

La méthode selon l'invention est plus spécifique et beaucoup plus sensible.

De surcroît la méthode selon l'invention est simple et reproductible tout en étant à moindre coût.

Le procédé selon l'invention permet d'être également non invasif et non douloureux.

La présente invention a pour but de remédier aux inconvénients précédemment évoqués. Un procédé ou méthode pour le diagnostic/pronostic/suivi d'un cancer chez les vertébrés, comprenant les étapes suivantes, est décrit:
i) extraction d'un matériel nucléique d'un échantillon biologique ;
ii) utilisation d'au moins une paire d'amorces d'amplification en vue d'obtenir des amplicons d'au moins une séquence cible ;
iii) utilisation d'au moins une sonde de détection pour détecter la présence desdits amplicons ;
caractérisé en ce que l'étape d'extraction est réalisée à partir d'un matériel nucléique extracellulaire d'un échantillon biologique.

Plus précisément ledit matériel nucléique correspond à l'ARN ou l'ADN du gène CA9 circulant à l'état libre dans un échantillon biologique.

Avantageusement, lorsque ledit matériel nucléique est de l'ARN, il subit une transcription inverse. De plus, dans ce cas, les étapes de transcription inverse et l'étape ii) sont effectuées en même temps (RT-PCR).

Ladite étape ii) selon l'invention est une PCR conventionnelle ou quantitative.

De manière avantageuse, les étapes ii) et iii) du procédé selon l'invention sont réalisées en même temps.

Ladite sonde de détection peut également comprendre un marqueur.

De manière plus précise, ladite paire d'amorces et/ou ladite sonde de détection comprend au moins 15 motifs nucléotidiques d'une séquence nucléotidique du gène CA9 choisie parmi les SEQ ID n° 1 à 6.

Sont aussi décrites:
- au moins une amorce d'amplification pour le diagnostic/pronostic/suivi d'un cancer comprenant au moins 15 motifs nucléotidiques d'une séquence nucléotidique choisie parmi les SEQ ID n°1 à 6 ;
- une sonde de détection pour le diagnostic/pronostic/suivi d'un cancer comprenant au moins 15 motifs nucléotidiques d'une séquence nucléotidique choisie parmi SEQ ID n°1 à 6.

Avantageusement, un kit pour le diagnostic/pronostic/suivi d'un cancer comprenant au moins une paire d'amorces citées ci-dessus et/ou au moins une sonde de détection citée ci-dessus est également décrit.

L'invention sera bien comprise à la lumière de la description qui suit, se rapportant à des exemples illustratifs, et en aucun cas exhaustifs, de la présente invention, en référence aux dessins, ci-joints, dans lesquels :
- la figure 1 illustre les taux d'ARNm CA9 de différents individus ;
- la figure 2 est un gel d'électrophorèse montrant les résultats d'une PCR conventionnelle ;
- la figure 3 représente les taux pré et post-opératoires d'ARNm CA9 chez des individus présentant un CRCC ;
- la figure 4 est une courbe ROC démontrant l'utilité diagnostique du taux d'ARNm CA9.

Une méthode, un procédé, un test et un kit permettant la mise en évidence d'un cancer, en vue du diagnostic, du pronostic ou du suivi thérapeutique, basés sur la mise en évidence d'un biomarqueur tumoral spécifique sur un simple prélèvement d'un échantillon biologique, sont décrits.

Un «échantillon biologique» est prélevé chez un individu en vue d'une quête de dépistage/diagnostic/suivi d'un cancer et notamment du rein. Cet échantillon, contenant des cellules et du matériel nucléique, peut être son sang, ses selles, sa salive, son urine, sa bile, son sérum, son plasma, son sperme, etc. ou un échantillon de tumeur.

Le «matériel nucléique» composé de séquences d'acides désoxyribonucléiques (ADN) ou ribonucléiques (ARN), ainsi prélevé chez ledit individu via ledit échantillon biologique comprend une séquence cible. Une «séquence cible» est une séquence nucléotidique spécifique d'un gène cible qu'il soit simple ou double brin, tel que le gène CA9.

Une «séquence nucléotidique» est un enchaînement de motifs nucléotidiques, c'est-à-dire une séquence d'acides nucléiques ou de polynucléotides ou de fragments de ces derniers.

On entend par «motif nucléotidique» un nucléotide naturel d'acide nucléique ou un nucléotide modifié (base, phosphate, sucre).

Les structures et les modifications de ces enchaînements sont soit naturelles, soit les résultantes d'une recombinaison génétique ou d'une synthèse chimique.

Les présents inventeurs ont axé leurs recherches sur le dosage des acides nucléiques d'un gène spécifique circulant à l'état libre dans un échantillon biologique, afin de créer un biomarqueur dans la détection d'un cancer.

Par exemple, la présence d'ARN ou d'ADN tumoral directement dans un échantillon biologique, telle l'analyse d'ARNm d'un gène dans le sérum d'un vertébré.

En effet, à titre illustratif, ils ont pu montrer que l'ARNm du gène CA9 pouvait être détecté directement dans le sérum ou le plasma d'un individu atteint d'un cancer rénal.

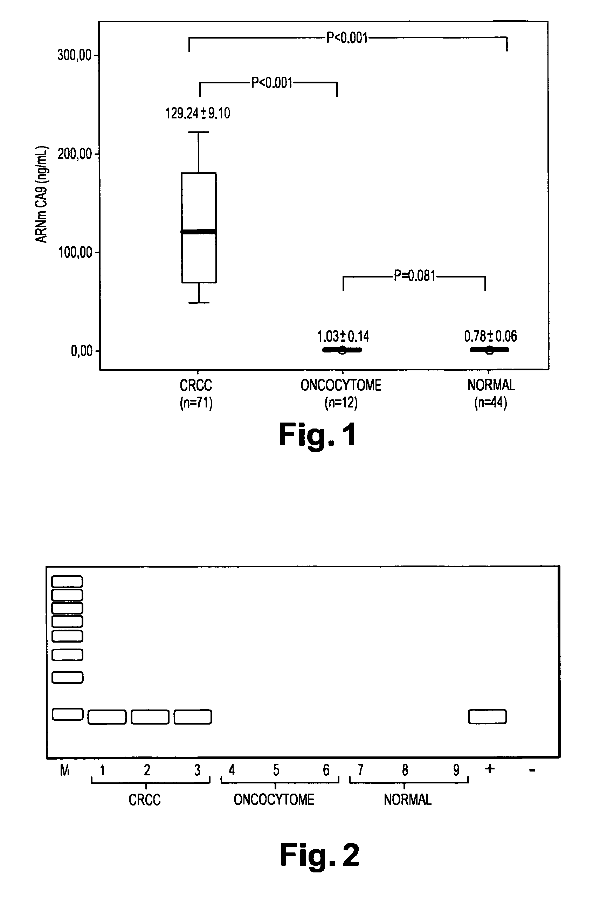

La figure 1 montre les taux d'ARNm CA9 chez des individus atteints de cancer du rein (CRCC), de tumeur bénigne (Oncocytome) et chez des individus sains. Ce schéma montre que les taux d'ARNm du gène CA9 chez les individus présentant un CRCC sont significativement plus élevés que chez les individus sains ou présentant une tumeur bénigne.

Jusqu'à présent, les acides nucléiques, tels les ADN ou ARN extracellulaires, étaient considérés comme trop fragiles et rapidement détruits par des enzymes ou par la congélation. Ainsi ils ne pouvaient résister dans de telles conditions, ce qui est un préjugé vaincu important.

Les présents inventeurs ont pu démontrer également la présence d'acides nucléiques du gène MN/CA9 dans des échantillons de sérum fraîchement recueilli, mais aussi après une simple coagulation du sang ou leur congélation.

Le procédé selon l'invention se rapporte à l'utilisation d'un nouveau marqueur moléculaire le gène CA9, et plus particulièrement ses acides nucléiques circulants extracellulaires dans un échantillon biologique.

Plus précisément il est recherché l'ARNm ou l'ADN du gène CA9 extracellulaire ou circulant à l'état libre, en opérant par une extraction de l'ARN ou l'ADN puis par une étape d'amplification (PCR conventionnelle ou quantitative) d'un échantillon biologique.

Il est possible d'utiliser d'autres méthodes pour quantifier l'ARNm ou l'ADN du gène CA9.

De plus, de nouvelles séquences nucléotidiques d'une séquence cible, et plus particulièrement celle du gène CA9, qui peuvent être utilisées en tant qu'amorces d'amplification ou de sondes d'hybridation afin de détecter et/ou pronostiquer et/ou suivre un individu atteint d'un cancer, sont également concernées.

Plus particulièrement, les inventeurs ont découvert que lesdites séquences nucléotidiques cibles extracellulaires étaient présentes dans des échantillons biologiques, prélevés de manière non invasive, chez des individus atteint d'un cancer, notamment de type rénal.

De manière plus précise, ladite séquence nucléotidique comprenant au moins 15 motifs nucléotidiques d'une séquence choisie parmi les SEQ ID n°1 à n°6 est très pertinente :
- en tant qu'amorce d'amplification pour amplifier les séquences cibles, tel que le gène CA9,
- en tant que sonde d'hybridation pour une hybridation spécifique sur des séquences cibles, tels que le gène CA9.

Selon la présente description, une «amorce d'amplification» est une séquence nucléique comprenant 15 à 200 motifs nucléotidiques, préférentiellement de 15 à 25 paires de bases d'au moins une séquence cible de matériel génétique.

Une «hybridation» est un processus par lequel deux séquences nucléiques, comme par exemple, une amorce et une séquence cible se lient dans des conditions spécifiques et bien connues de l'homme du métier.

Une «sonde d'hybridation» est une séquence nucléique de 15 à 200 motifs nucléotidiques, préférentiellement de 100 à 190 paires de bases d'au moins une séquence cible de matériel génétique. Cette sonde possède une spécificité d'hybridation dans des conditions spécifiques et déterminées afin qu'elle s'hybride avec la séquence nucléique cible.

L'ARNm du gène cible CA9 a pour séquence (genebank accession number: NM 001216):

### i) La 1^{ère} étape consiste en une extraction du matériel nucléique d'un échantillon biologique.

La mise en évidence de la présence d'ARN ou d'ADN tumoral, à partir d'un échantillon biologique, nécessite l'extraction de l'ARN ou de l'ADN total dudit échantillon.

Cette extraction est réalisée par tout protocole d'extraction d'acides nucléiques d'échantillon biologique de type connu en soi. Cette étape est une purification consistant à séparer et à concentrer les acides nucléiques des autres constituants.

Par exemple, l'échantillon biologique peut être du sang (5ml) qui est ensuite centrifugé à 1200 g pendant 10 minutes à 4°C. Ainsi le sérum est séparé avant l'extraction de l'ARN total.

L'extraction d'ARN total peut être effectuée en utilisant par exemple le kit «RNeasy de Qiagen» conformément aux recommandations du fournisseur.

Les échantillons d'ARN sont ensuite stockés à -80°C .

Bien entendu, l'homme de l'art sait adapter l'extraction, la purification et la conservation des acides nucléiques en fonction des échantillons biologiques dont ils sont issus.

Comme il est recherché des acides nucléiques circulant à l'état libre, c'est-à-dire à extracellulaires, il n'est pas nécessaire de réaliser une lyse des cellules tumorales circulantes. Ce qui résulte en un procédé plus simple, moins couteux en termes de temps et d'argent.

**S'il s'agit de dosage d'ARN, l'étape i-bis) consiste en une transcription inverse.**

L'étape de transcription inverse consiste à synthétiser un brin d'ADN complémentaire (ADNc) à partir d'ARNm. La synthèse d'un ADNc double brin, résultante d'une amplification ultérieure, permet de cloner des gènes souhaités afin de les utiliser comme sondes nucléotidiques pour la détection ou le dosage des ARNm correspondants.

Après purification, les ARNm subissent une étape de transcription inverse en ADNc simple brin en utilisant une enzyme à activité réverse transcriptase. Cette étape peut être réalisée à l'aide de kits commerciaux comme le kit « SuperScript^{™} First-Strand Synthesis System for RT-PCR » commercialisé par Invitrogen ou par toute méthode connue en soi.

### ii) La 2^{e} étape est l'amplification par PCR (Polymerase Chain Reaction) de l'ADN ou de l'ADNc.

Le processus d'amplification par polymérisation enzymatique (technique de réplication ciblée, in vitro, dite «PCR», Polymerase Chain Reaction), permet d'obtenir à partir d'un échantillon contenant de l'ADN ou de l'ADNc, d'importantes quantités d'un fragment d'ADN spécifique, tel un marqueur tumoral, et de longueur définie en utilisant une paire d'amorces nucléotidiques.

Cette étape se réalise via l'utilisation d'amorces d'amplification afin de générer des amplicons d'au moins une séquence cible du matériel nucléique.

Ladite amorce et/ou ladite sonde comprend au moins 15 motifs nucléotidiques d'une séquence choisie parmi :
- une séquence de SEQ ID n°1 à 6 ;
- une séquence homologue de SEQ ID n°1 à 6 complémentaire ou suffisamment complémentaire ; ou présentant une homologie suffisante pour s'hybrider à SEQ ID n°1 à 6 ou à le urs séquences complémentaires ;
- une séquence comprenant une séquence de SEQ ID n°1 à 6, qui aurait la même fonction que l'amorce d'amplification.

Les séquences d'amorces et/ou des sondes comprenant au moins 15 motifs nucléotidiques d'une séquence nucléotidique du gène CA9 sont choisies parmi :
- GGA CAA AGA AGG GGA TGA CC (SEQ ID N°1)
- AGT TCT GGG AGC GGC GGG A (SEQ ID N°2)
- CAG AGT CAT TGG CGC TAT GGA (SEQ ID N°3)
- CGA GCT GGG GGC GGA TA (SEQ ID N°4)
-
-

Ces amorces sont conçues en vue de recouvrir la jonction d'épissage afin d'éliminer les signaux engendrés par une contamination génomique.

A titre illustratif, la PCR conventionnelle peut être réalisée à l'aide du kit «Eppendorf MasterMix», selon les recommandations du fournisseur ou par toute méthode connue en soi.

Par exemple, les amorces utilisées de la séquence cible CA9 (186 pb) sont les SEQ ID N°1 et 2.

Le cycle PCR suivant peut être utilisé pour réaliser l'amplification génique de toute séquence spécifique à l'aide d'un thermocycleur (« Perkin Elmer » par exemple).

Après une étape préalable de dénaturation à 94°C (2 minutes), trente cinq cycles PCR peuvent ainsi être réalisés afin d'amplifier suffisamment le matériel génique. Chaque cycle pourra comprendre une nouvelle étape de dénaturation à 94°C (1 minute) suivie d'une étape d ite d'hybridation des amorces à 57°C (1 minute) et enfin une dernière éta pe dite d'élongation à 72°C (1 minute pour 1000 nucléotides à amplifier). Une étape finale à 72°C (5 minutes) est également nécessaire afin de terminer l'élongation de tous les fragments. La température d'hybridation des amorces devra être calculée en fonction du Tm de chaque amorce.

De manière alternative, lorsqu'il s'agit d'analyser l'ARNm issu d'un échantillon biologique, la transcription inverse et la PCR (RT-PCR) sont réalisées simultanément en une étape (étapes i-bis et ii simultanées). La RT-PCR en une étape est effectuée en utilisant par exemple, les kits «Super Script^{™} One-Step RT-PCR» et «Platinum tag » de la société Invitrogen conformément aux recommandations du fournisseur. La réaction de RT-PCR sera également réalisée sur un thermocycleur (« Perkin Elmer » par exemple) selon les conditions suivantes : une étape à 50°C (30 min) permettant la transcription inverse suivie d'une étape de dénaturation à 94°C (2 min), puis suivie de 35 cycles de PCR comme décrit précédemment.

Pour chaque analyse PCR, des contrôles négatifs (sans acides nucléiques) et positifs (par exemple à partir d'un plasmide codant le gène CA9) sont réalisés en parallèle.

Avantageusement, la PCR opérée peut être quantitative. Ainsi la technique «SYBR green» est utilisée et basée sur la courbe standard obtenue à partir de plasmides codant le gène CA9.

Dans ce cas, les amorces de la séquence cible CA9 (100bp) peuvent être les SEQ ID N°3 et 4.

La PCR quantitative est effectuée après la réaction de RT-PCR décrite précédemment à l'aide du kit «QuantiTect SYBR Green PCR Master Mix» de la société Quiagen conformément aux recommandations du fournisseur. Les conditions de la réaction PCR réalisée à l'aide d'un thermocycleur (par exemple « Perkin Elmer ») sont les suivantes : une étape de dénaturation à 95°C (10 minutes), suivie de quarante cycles constitués d'une nouvelle étape de dénaturation à 95°C (15 secondes), d'une étape d'hybridation à 58°C (15 secondes) et d'une étape d'élongation à 72°C (30 secondes). Un cycle final constitué d'une étape de dénaturation à 95°C (15 secondes), d'une étape d'él ongation finale à 60°C (1 minute) et d'une dernière dénaturation à 95°C (1 5 secondes) doit être réalisée conformément aux recommandations du fournisseur.

### iii) La 3^{e} étape est la détection.

Lors de l'étape de détection des acides nucléiques cibles, il peut être prévu d'utiliser une sonde de détection spécifique.

Bien entendu, cette étape de détection directe ou indirecte, peut être réalisée par tout autre mode de détection de type connu en soi.

De manière avantageuse, la sonde d'hybridation est une sonde de détection. En effet, la sonde d'hybridation peut comprendre un marqueur permettant sa détection. Ledit «marqueur» est un traceur capable de repérer, marquer ou distinguer ce qui est reconnaissable du fait de sa propriété physique ou chimique (radioactivité, fluorescence, masse, couleur, luminescence et autres... via des détections optique, électrique, etc.) en très faible quantité.

Les amorces fonctionnelles peuvent être analysées avec un fluorochrome ou un autre fluorescent ou «quencher» qui se lie spécifiquement au produit d'amplification (ADN double brin).

Ainsi on peut détecter une réaction d'hybridation entre une sonde de détection et la séquence cible.

De manière alternative, il peut être prévu que la sonde de détection soit une sonde de détection «molecular beacon».

La sonde d'hybridation peut également être une sonde de capture, où ladite sonde est immobilisée sur un support solide par tout moyen approprié (de type connu en soi). On détectera ensuite la réaction d'hybridation entre la sonde de capture et la séquence cible.

Pour la détection de la réaction d'hybridation, on peut également utiliser des séquences cibles marquées, directement ou indirectement de la séquence cible.

De manière alternative, l'étape d'hybridation peut être une étape de marquage et/ou de clivage de la séquence cible.

Afin de s'affranchir des dimères d'amorces qui se constituent spontanément, il peut être prévu de lier aux amorces sens et antisens une sonde taqman spécifique construite à l'aide de logiciels bien connus.

Les inventeurs de la présente invention, en vue de corriger toute variabilité d'efficacité enzymatique possible, ont normalisé l'expression du gène cible par la détermination d'un ratio gène-cible/gène de ménage (NADPH ou β-actine par exemple) dont l'expression est obligatoire et courante chez tous les individus. Les amorces du gène de ménage et plus particulièrement le NADPH sont par exemple:
- amorce sens 5' AAA GGA CAT TTC CAC CGC AAA 3'
- amorce antisens 5' GGT CGG GTC AAC GCT AGG CT 3'

De manière alternative, l'amplification, étape ii), (PCR conventionnelle ou quantitative) est réalisée en même temps que l'étape de la détection, étape iii).

A titre d'exemple, les produits de PCR ou amplicons peuvent être séparés par électrophorèse sur gel d'agarose 1.0%, puis visualisés par illumination sous UV après coloration de l'ADN par le bromure d'éthidium.

Le fragment attendu est identifié par comigration d'un marqueur de taille moléculaire.

La figure 2 montre ainsi la positivité de l'ARNm CA9 dans les cas de CRCC et la négativité dans les tumeurs bénignes (oncocytome) et les individus sains. M signifie marqueur de taille moléculaire.

Les résultats explicités ci-après sont illustratifs d'expériences de comparaison réalisées et en aucun cas limitatifs.

**Tableau 1: Caractéristiques des individus**

| | No. de cas (%) | | |
|---|---|---|---|
| Patients avec CRCC (N=71) | | | |
| Sexe | | | |
| | Masculin | 45 | 63.4% |
| | Feminin | 26 | 36.6% |

| Age ( année ) | | | |
|---|---|---|---|
| | Limite | 35 - 86 | |
| | Médian | 66 | |

| TNM | | | |
|---|---|---|---|
| | T1 | 30 | 42.3% |
| | T2 | 5 | 7.0% |
| | T3 | 36 | 50.7% |

| Grade | | | |
|---|---|---|---|
| | G1 | 12 | 16.9% |
| | G2 | 32 | 45.1% |
| | G3 | 22 | 31.0% |
| | G4 | 5 | 7.0% |
| Patients avec Oncocytome | | (N=12) | |

| Sexe | | | |
|---|---|---|---|
| | Masculin | 4 | 33.4% |
| | Feminin | 8 | 66.6% |

| Age ( année ) | | | |
|---|---|---|---|
| | Limite | 27-76 | |
| | Médian | 58 | |
| Individuelles sans tumeur | | (N=44) | |

| Sexe | | | |
|---|---|---|---|
| | Mascuim | 36 | 81.8% |
| | Féminin | 8 | 18.2% |

| Age ( année ) | | | |
|---|---|---|---|
| | Limite | 50 - 80 | |
| | Médian | 64 | |

**Tableau 2 : PCR quantitative des différents groupes**

| | | No. de cas | CA9 (ng/mL) | P- valeur |
|---|---|---|---|---|
| Patients | | | | |
| Age | | | | 0.858 |
| | ≤ 50 | 11 | 123.32 ± 25.64 | |
| | 51-69 | 33 | 130.79 ± 11.95 | |
| | ≥ 70 | 27 | 129.81 ± 16.36 | |
| Sexe | | | | 0.535 |
| | Masculin | 45 | 131.99 ± 11.03 | |
| | Féminin | 26 | 124.47 ± 16.16 | |
| TNM | | | | 0.040 |
| | T1 | 30 | 137.53 ± 14.03 | |
| | T2 | 5 | 113.23 ± 22.42 | |
| | T3 | 36 | 194.81 ± 12.53 | |
| Grade | | | | 0.691 |
| | 1 | 12 | 154.02 ± 26.68 | |
| | 2 | 32 | 123.22 ± 13.18 | |
| | 3 | 22 | 118.47 ± 13.39 | |
| | 4 | 5 | 155.71 ± 48.91 | |
| Taille de tumeur | | | | 0.445 |
| | < 4 cm | 23 | 131.65 ± 15.83 | |
| | 4-8 cm | 32 | 136.24 ± 13.83 | |
| | >8 cm | 16 | 111.80 ± 19.22 | |

| Patients avec Oncocytome | | | | |
|---|---|---|---|---|
| Sexe | | | | 0.416 |
| | Masculin | 4 | 1.18 ± 0.15 | |
| | Féminin | 8 | 0.96 ± 0.29 | |

| Individuelles sans tumeur | | | | |
|---|---|---|---|---|
| Sexe | | | | 0.540 |
| | Masculin | 36 | 0.81 ± 0.07 | |
| | Féminin | 8 | 0.87 ± 0.17 | |

Le Tableau 1, ci-dessous, décrit les caractéristiques des individus de l'étude, à titre d'exemple, pour la présente invention. 71 individus présentant un CRCC, 12 individus avec un oncocytome rénal et 44 individus sains.

Le Tableau 2 ci-dessus, donne les résultats d'une PCR quantitative dans les différents groupes. Les résultats indiquent que les taux d'ARNm CA9 ne sont pas liés à la taille de la tumeur.

**Tableau 3 : Comparaison des tests selon l'invention et ELISA (« Human Carbonic Anhydrase IX ; R&D Systems ») :**

| | RT-PCR en une étape | PCR Quantitative | ELISA |
|---|---|---|---|
| Sensibilité | 93.0% | 91.5% | 34.8% |
| Spécificité | 100.0% | 100.0% | 89.6% |
| Valeur prédictive positive | 100.0% | 100.0% | 86.5% |
| Valeur prédictive négative | 89.8% | 88.0% | 41.7% |

Le Tableau 3 ci-dessus compare les résultats du test de détection selon l'invention d'ARNm du gène CA9 dans le sérum/plasma avec celui du test ELISA utilisant la protéine CA9 dans le sérum pour le diagnostic de cancer.

Dans ce but, un seuil de positivité de la technique ELISA a été au préalable calculé sur une population de 48 individus sains. Ce seuil de positivité est de 100 pg/ml et correspond classiquement à la moyenne des échantillons plus ou moins deux déviations standards (+/- 2DS). Les résultats de la technique de détection des ARNm CA9 sont bien meilleurs que ceux du test ELISA en termes de sensibilité, de spécificité et de valeur prédictive.

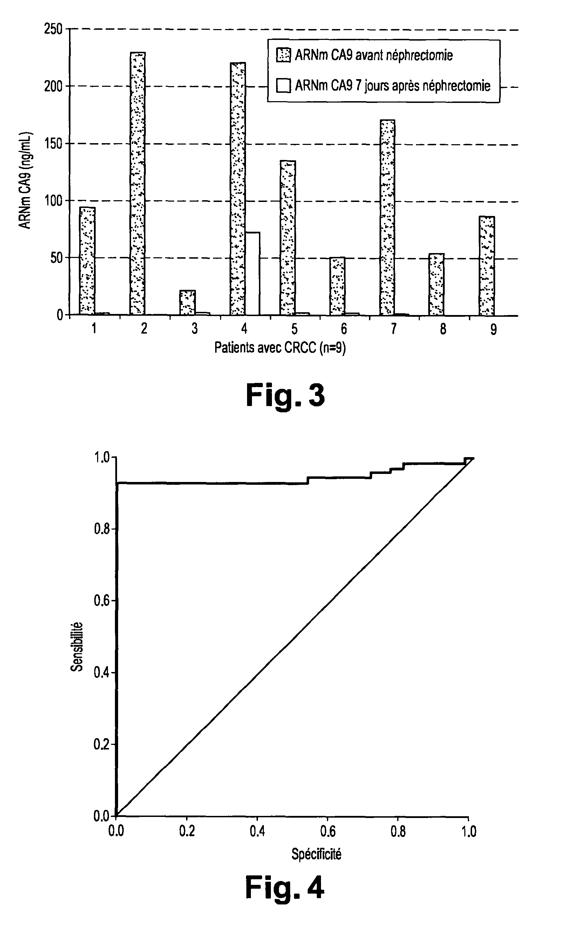

La figure 3 montre la diminution du taux d'ARNm CA9 après chirurgie (néphrectomie). En effet, les taux d'ARNm CA9 diminuent significativement ou s'annulent une semaine après l'ablation de la tumeur.

La figure 4 est la courbe ROC « Receiver Operating Characteristic » démontrant l'utilité diagnostique du taux d'ARNm CA9. Cette courbe permet d'analyser les modifications de la spécificité et de la sensibilité d'un test pour différentes valeurs du seuil de discrimination. L'aire sous la courbe ROC est un estimateur de l'efficacité globale. Dans le présent cas selon l'invention, l'aire sous la courbe est de 0.946 (intervalle de confiance 95% : 0,904-0,993) ce qui signifie que le test est parfaitement discriminatif.

Comparé aux tests actuellement commercialisés, le test décrit diffère par l'utilisation d'un nouveau marqueur moléculaire et plus particulièrement, les acides nucléiques du gène CA9 extracellulaires, avec une technique d'amplification et/ou détection et/ou de quantification (PCR ou RT PCR quantitative ou conventionnelle), d'un procédé non invasif tout en ayant un bon ratio coût/efficacité.

Bien entendu, la méthode et le test décrits peuvent être combinés avec, ou inclure, d'autres marqueurs moléculaires en vue d'augmenter encore sa sensibilité et sa spécificité en fonction des cancers recherchés.

La présente invention permet, en outre, d'améliorer la stratégie de dépistage ainsi que les traitements des formes précoces de cancer ou précancéreuses.

## Revendications

1. Procédé ou méthode pour le diagnostic/pronostic/suivi d'un cancer chez les individus, comprenant les étapes suivantes :
i) extraction d'un matériel nucléique extracellulaire d'un échantillon biologique, ledit échantillon pouvant être du sang, du sérum ou du plasma;
ii) utilisation d'au moins une paire d'amorces d'amplification en vue d'obtenir des amplicons d'au moins une séquence cible ;
iii) utilisation d'au moins une sonde de détection pour détecter la présence desdits amplicons ;
**caractérisé en ce que** ledit matériel nucléique extracellulaire correspond à l'ARN du gène CA9 circulant à l'état libre dans ledit échantillon biologique.

2. Procédé selon la revendication 1, **caractérisé en ce que** lorsque ledit matériel nucléique est de l'ARN, il subit une transcription inverse.

3. Procédé selon la revendication 2, **caractérisé en ce que** les étapes de transcription inverse et l'étape ii) sont effectuées en même temps (RT PCR).

4. Procédé selon l'une des revendications 1 ou 3, **caractérisé en ce que** ladite étape ii) est une PCR conventionnelle ou quantitative.

5. Procédé selon l'une des revendications 1, 3 ou 4, **caractérisé en ce que** les étapes ii) et iii) sont réalisées en même temps.

6. Procédé selon l'une des revendications précédentes, **caractérisé en ce que** ladite sonde de détection comprend un marqueur.

7. Procédé selon l'une des revendications précédentes, **caractérisé en ce que** ladite paire d'amorces et/ou ladite sonde de détection comprend au moins 15 motifs nucléotidiques d'une séquence nucléotidique du gène CA9 choisie parmi les SEQ ID n ° 1 à 6.

8. Utilisation d'au moins une paire d'amorces, chaque amorce comprenant au moins 15 motifs nucléotidiques d'une séquence nucléotidique choisie parmi les SEQ ID n °1 à 6, dans l'amplification d'une séquence ARN extracellulaire du gène CA9 circulant à l'état libre dans un échantillon biologique choisi parmi le sang, le sérum ou le plasma, pour le diagnostic/pronostic/suivi d'un cancer.

9. Utilisation d'au moins une sonde comprenant au moins 15 motifs nucléotidiques d'une séquence nucléotidique choisie parmi SEQ ID n ° 1 à 6 dans la détection d'une séquence ARN extracellulaire du gène CA9 circulant à l'état libre dans un échantillon biologique choisi parmi le sang, le sérum ou le plasma, pour le diagnostic/pronostic/suivi d'un cancer.

## Claims

1. A process or method for the diagnosis/prognosis/follow-up of cancer in individuals, comprising the following steps:
i) extracting extracellular nucleic material from a biological sample, the said sample being possibly blood, serum or plasma;
ii) using at least one pair of amplification primers in order to obtain amplicons of at least one target sequence;
iii) using at least one detection probe to detect the presence of the said amplicons;
**characterised in that** the said extracellular nucleic material is the RNA of the CA9 gene circulating in the free state in the said biological sample.

2. A process according to claim 1, **characterised in that** when the said nucleic material is RNA, it undergoes reverse transcription.

3. A process according to claim 2, **characterised in that** the steps of reverse transcription and step ii) are carried out at the same time (RT PCR).

4. A process according to one of claims 1 or 3, **characterised in that** the said step ii) is conventional or quantitative PCR.

5. A process according to one of claims 1, 3 or 4, **characterised in that** the steps ii) and iii) are carried out at the same time.

6. A process according to one of the preceding claims, **characterised in that** the said detection probe comprises a marker.

7. A process according to one of the preceding claims, **characterised in that** the said pair of primers and/or the said detection probe comprises at least 15 nucleotide patterns of a nucleotide sequence of the CA9 gene selected from SEQ ID nos. 1 to 6.

8. Use of at least one pair of primers, each primer comprising at least 15 nucleotide patterns of a nucleotide sequence selected from SEQ ID nos. 1 to 6, in the amplification of an extracellular RNA sequence of the CA9 gene circulating in the free state in a biological sample selected from blood, serum or plasma, for the diagnosis/prognosis/follow-up of cancer.

9. Use of at least one probe comprising at least 15 nucleotide patterns of a nucleotide sequence selected from SEQ ID nos. 1 to 6 in the detection of an extracellular RNA sequence of the CA9 gene circulating in the free state in a biological sample selected from blood, serum or plasma, for the diagnosis/prognosis/follow-up of cancer.

## Patentansprüche

1. Verfahren oder Methode für die Diagnose, Prognose, Kontrolle eines Krebses bei Patienten mit folgenden Schritten:
i) Extraktion von extrazellulärer Nucleinsubstanz aus einer biologischen Probe, wobei es sich dabei um eine Blut-, Serum- oder Plasmaprobe handeln kann;
ii) Anwendung von mindestens einem Amplifikations-Primer-Paar, um Amplikone von mindestens einer Target-Sequenz zu erzielen;
iii) Anwendung von mindestens einer Detektionssonde, um vorhandene Amplikone zu erkennen;
**dadurch gekennzeichnet, dass** die extrazelluläre Nucleinsubstanz der RNA des CA9 Gens entspricht, das in der biologischen Probe frei zirkuliert.

2. Verfahren nach Anspruch 1, **dadurch gekennzeichnet, dass** wenn es sich bei der Nucleinsubstanz um RNA handelt, diese einer inversen Transkription unterzogen wird.

3. Verfahren nach Anspruch 2, **dadurch gekennzeichnet, dass** die Schritte der inversen Transkription und der Schritt ii) gleichzeitig stattfinden (RT PCR).

4. Verfahren nach einem der Ansprüche 1 oder 3, **dadurch gekennzeichnet, dass** der Schritt ii) eine konventionelle oder quantitative PCR ist.

5. Verfahren nach einem der Ansprüche 1, 3 oder 4, **dadurch gekennzeichnet, dass** die Schritte ii) und iii) gleichzeitig stattfinden.

6. Verfahren nach einem der vorausgehenden Ansprüche, **dadurch gekennzeichnet, dass** die Detektionssonde einen Marker umfasst.

7. Verfahren nach einem der vorausgehenden Ansprüche, **dadurch gekennzeichnet, dass** das Primer-Paar und/oder die Detektionssonde mindestens 15 Nucleotidmuster einer unter den SEQ ID Nr. 1 bis 6 ausgewählten Nucleotidsequenz des CA9 Gens umfasst.

8. Anwendung von mindestens einem Primer-Paar, wobei jeder Primer mindestens 15 Nucleotidmuster einer unter den SEQ ID Nr. 1 bis 6 ausgewählten Nucleotidsequenz umfasst, bei der Amplifikation einer extrazellulären RNA-Sequenz des CA9 Gens, das frei in einer biologischen Probe zirkuliert, wobei es sich bei der Probe um eine Blut-, Serum- oder Plasmaprobe für die Diagnose/Prognose oder Kontrolle von Krebs handelt.

9. Anwendung von mindestens einer Sonde mit mindestens 15 Nucleotidmustern einer unter den SEQ ID Nr. 1 bis 6 ausgewählten Nucleotidsequenz bei der Detektion einer extrazellulären RNA-Sequenz des CA9 Gens, das frei in einer biologischen Probe zirkuliert, wobei es sich bei der Probe um eine Blut-, Serum- oder Plasmaprobe für die Diagnose/Prognose oder Kontrolle von Krebs handelt.
